# EUROPEAN PATENT APPLICATION

(11) **EP 2 762 192 A1**
(43) Date of publication of application: **06.08.2014**
(21) Application number: 14152294.6
(22) Date of filing: 23.01.2014
(51) Int. Cl.: A61M 25/10

(54) **Balloon catheter**

(30) Priority: 01.02.2013 JP 2013018762
(71) Applicant: ASAHI INTECC CO., LTD., Nagoya-shi, Aichi 463-0024 (JP)
(72) Inventor: Katsurada, Takeharu, Nagoya-shi, Aichi 463-0024 (JP); Yagi, Takayuki, Nagoya-shi, Aichi 463-0024 (JP)
(74) Representative: Winter, Brandl, Fürniss, Hübner, Röss, Kaiser, Polte - Partnerschaft

(57) **Abstract**

When folded, a balloon (20) in a balloon catheter (10) has a distal end section (24) which has a small outer diameter (D1) and in which a gap (213) between a body part (210) and a wing part (211) is narrow, and a proximal end section (25) which has a large outer diameter (D2) and in which a gap (214) between the body part (210) and a wing part (212) is wide.

## Description

### BACKGROUND OF THE INVENTION

### 1. Field of the Invention

The present invention relates to a balloon catheter to be inserted in a narrowed part of a blood vessel to dilate the narrowed part.

### 2. Description of the Related Art

A balloon catheter is known as an example of a catheter that is inserted in a narrowed part of a blood vessel and is inflated for medical treatment. A balloon catheter includes a balloon to be inflated, an outer shaft, and an inner shaft inserted in the outer shaft. The inner shaft is used such that a guide wire extends therethrough. The outer shaft is used such that liquid for inflating the balloon (for example, a contrast agent or physiologic saline solution) is carried through a lumen provided between the outer shaft and the inner shaft.

The balloon catheter is inserted to a narrowed part of a blood vessel, for example, by using a guide wire or a guide catheter, and the balloon is inflated at the narrowed part to ensure the bloodstream. If the balloon does not pass through the narrowed part, medical treatment cannot be performed. For this reason, the balloon catheter is inserted in the blood vessel with the balloon being folded to a small outer diameter.

For example, Japanese Unexamined Patent Application Publication No. 8-98892 discloses a method for folding a balloon. In this method, after the balloon is wound around an outer periphery of an inner shaft under reduced pressure, it is entirely covered with a heat-shrinkable tube, and the heat-shrinkable tube is shrunk by a heater. However, since a marker having rigidity is provided in the balloon of the disclosed balloon catheter, if the heat-shrinkable tube is shrunk by strong force to fold the balloon, the balloon may be broken by the marker. For this reason, it is difficult to reduce the outer diameter of the balloon.

Accordingly, U.S. Patent No. 6,540,721 discloses a balloon catheter in which a balloon is strongly folded while an inner shaft is doped with a radiopaque material, such as tungsten or bismuth, without using any marker having rigidity. In this disclosed balloon catheter, insertability of the balloon to the narrowed part is high because the outer diameter of the balloon can be made small. However, since not only a distal end portion but also a proximal end portion of the balloon is strongly folded, liquid for inflating the balloon is unlikely to enter the balloon, and the inflation speed of the balloon is low.

### SUMMARY OF THE INVENTION

The present invention has been made in view of the above circumstances, and it is an object of the invention to provide a balloon catheter that ensures insertability of a balloon to a narrowed part and that increases an inflation speed and a deflation speed of the balloon.

The above object is achieved by a balloon catheter in accordance with claim 1 or a method for folding such a balloon in accordance with claim 8. Claims 2 to 7 refer to embodiments of the balloon catheter.

A balloon catheter according to the present invention includes a balloon including a body part and a wing part, an outer shaft connected to a proximal end of the balloon, and an inner shaft inserted in the outer shaft and connected to a distal end of the balloon. When the balloon is folded, the balloon has a distal end section having a first outer diameter and a proximal end section having a second outer diameter larger than the first outer diameter, and a gap between the body part and the wing part in the distal end section is less than a gap between the body part and the wing part in the proximal end section.

The balloon catheter according to the present invention includes, when the balloon is folded, the distal end section in which an outer diameter is small and a gap between the body part and a wing part is narrow and the proximal end section in which an outer diameter is large and a gap between the body part and a wing part is wide. Hence, a narrowed part can be dilated by the proximal end section with the large diameter by pushing in the balloon catheter with the distal end section having the small outer diameter being caught by the narrowed part. Further, since the gap between the body part and the wing part in the proximal end section of the balloon is wide, liquid for inflating the balloon can easily flow into the balloon, and this can increase the inflation speed. Still further, since the balloon stores the shape such that the gap between the body part and the wing part is narrow in the distal end section of the balloon, when the balloon is returned from an inflated state to a folded state, the wing part of the distal end section quickly returns to a position where the gap to the body part is narrow. This allows the liquid to be swiftly collected from the distal end of the balloon, and therefore, increases the deflation speed.

### BRIEF DESCRIPTION OF THE DRAWINGS

Fig. 1 is an overall view of a balloon catheter according to an embodiment;
Fig. 2 is a side view of an inflated balloon (an enlarged view of a section II in Fig. 1);
Figs. 3A to 3D illustrate a folded state of the balloon, Fig. 3A is a side view of the balloon in the folded state, Fig. 3B is a cross-sectional view of a distal end section of the balloon in the folded state, Fig. 3C is a cross-sectional view of a proximal end section of the balloon in the folded state, and Fig. 3D illustrates an outer diameter of the balloon in the folded state;
Figs. 4A to 4D illustrate a process for folding up the balloon; and
Figs. 5A and 5B illustrate a balloon catheter according to another embodiment, Fig. 5A is a side view of a balloon in an inflated state, and Fig. 5B is a side view of the balloon in a folded state.

### DESCRIPTION OF THE PREFERRED EMBODIMENTS

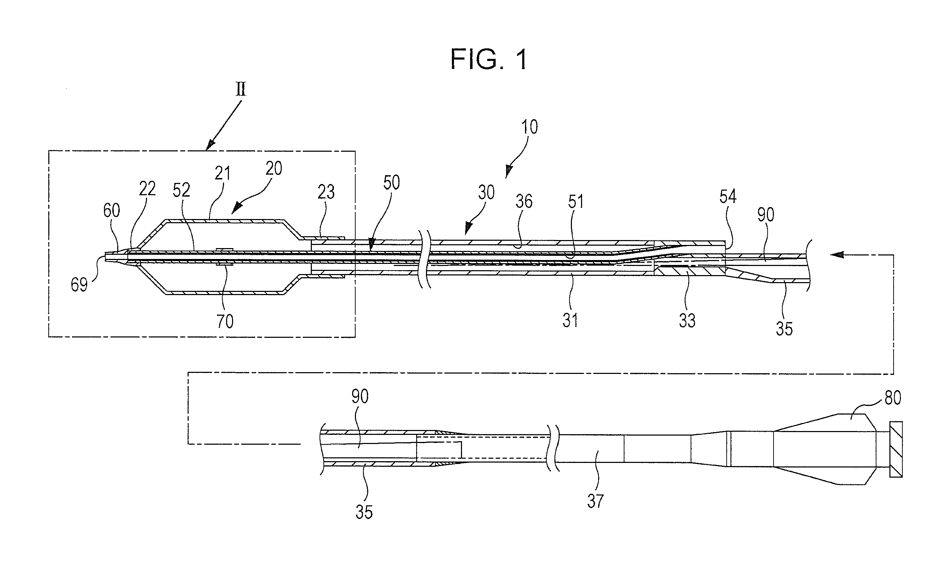

A balloon catheter 10 according to an embodiment will now be described with reference to Figs. 1 to 5B. In Figs. 1 to 3A and 4A to 5B, a left side is a distal end side to be inserted in the body, and a right side is a proximal end side to be manipulated by a manipulator such as a doctor. For easy understanding, elements that are relatively smaller than the other elements, for example, wing parts 211 and 212 included in a balloon 20 and gaps 213 and 214 provided between a body part 210 of the balloon 20 and the wing parts 211 and 212 are illustrated in a slightly exaggerated manner in a dimensional relationship with the other elements.

For example, the balloon catheter 10 is used to treat a narrowed part in a blood vessel of the heart. As illustrated in Fig. 1, the balloon catheter 10 mainly includes a balloon 20, an outer shaft 30, an inner shaft 50, a tip 60, and a connector 80.

The balloon 20 is formed by a resin member, and includes a dilating part 21 for dilating the narrowed part, a distal-end attachment part 22 disposed at a distal end, and a proximal-end attachment part 23 disposed at a proximal end. The distal-end attachment part 22 is fixed to an extending portion 52 of a below-described inner shaft 50 with a tip 60 being disposed therebetween. The proximal-end attachment part 23 is fixed to an outer peripheral surface of a distal end portion of an outer shaft 30.

The outer shaft 30 is a tubular member that constitutes an inflation lumen 36 through which fluid is supplied to inflate the balloon 20. The outer shaft 30 includes, in order from the distal end, a distal-end outer shaft part 31, a joint part 33, a middle outer shaft part 35, and a proximal-end outer shaft part 37. The distal-end outer shaft part 31 and the middle outer shaft part 35 are tubes formed of resin such as polyamide, polyamide elastomer, polyolefin, polyester, or polyester elastomer. The joint part 33 is where the distal-end outer shaft part 31, the middle outer shaft part 35, and the inner shaft 50 are joined by welding.

The proximal-end attachment part 23 of the balloon 20 is fixed to an outer periphery of a distal end portion of the distal-end outer shaft part 31. The inner shaft 50 is disposed in the distal-end outer shaft part 31, and the inflation lumen 36 is provided between the distal-end outer shaft part 31 and the inner shaft 50.

The proximal-end outer shaft part 37 is a metallic tubular member called a hypotube. A distal end portion of the proximal-end outer shaft part 37 is fixedly inserted in a proximal end portion of the middle outer shaft part 35. To a proximal end portion of the proximal-end outer shaft part 37, the connector 80 is attached. When liquid, such as a contrast agent or physiologic saline solution, is supplied from an indeflator (not illustrated) attachable to the connector 80 in order to inflate the balloon 20, it flows through the inflation lumen 36 and inflates the balloon 20. The material of the proximal-end outer shaft part 37 is not particularly limited. In this embodiment, the proximal-end outer shaft part 37 is formed of stainless steel (SUS 304). As another material, a superelastic alloy, such as a Ni-Ti alloy, may be used.

A core wire 90 is attached to an inner peripheral surface of the distal end portion of the proximal-end outer shaft part 37. The core wire 90 is a metallic wire of circular cross section. The core wire 90 has a tapered shape with a diameter decreasing toward a distal end. The material of the core wire 90 is not particularly limited. For example, stainless steel (SUS 304), a superelastic alloy such as a Ni-Ti alloy, or a piano wire can be used.

A proximal end portion of the core wire 90 is fixed to the distal end portion of the proximal-end outer shaft part 37 by brazing or laser welding. The core wire 90 extends through the middle outer shaft part 35 and the joint part 33 and reaches the distal end portion of the distal-end outer shaft part 31. The core wire 90 is fixed at the joint part 33. When push-in force or rotational force acts on the core wire 90, it is transmitted to the distal-end outer shaft part 31 and the inner shaft 50 via the joint part 33.

A proximal end of the inner shaft 50 is welded to the joint part 33 of the outer shaft 30 to form a proximal-end guide wire port 54.

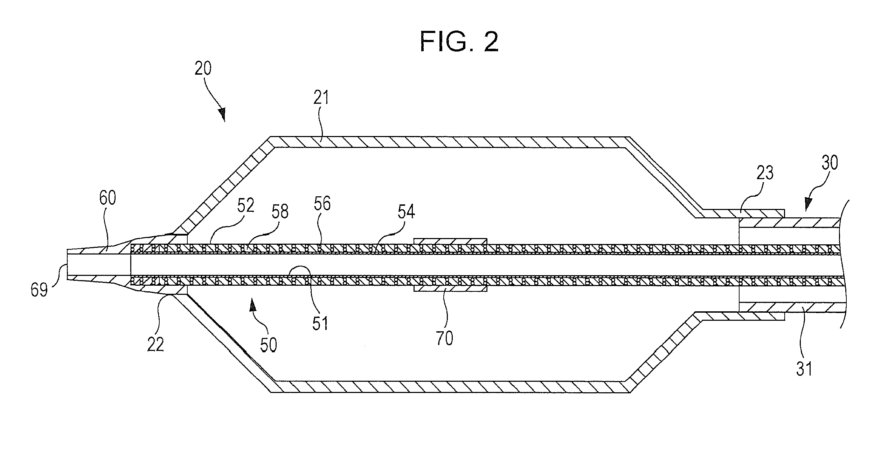

As illustrated in Fig. 2, the inner shaft 50 includes, in order from a radial inner side, an inner layer 54, a braid 56 serving as a reinforcement, and an outer layer 58.

The inner layer 54 is a tubular member formed of resin, and contains a guide wire lumen 51 in which a guide wire is to be inserted. The resin material that forms the inner layer 54 is not particularly limited, and polytetrafluoroethylene (PTFE) is used in this embodiment.

The braid 56 serving as a reinforcement is disposed on an outer periphery of the inner layer 54. The braid 56 is formed by weaving a plurality of strands in a net form (mesh form). In this embodiment, every two of eight strands in one of right-handed and left-handed directions are woven alternately over and under every two of eight strands in the other direction to weave 16 strands in total into a mesh (a combination of 8 strands x 8 strands).

However, the combination of the number of strands that constitute the braid 56 is not limited to the combination of 8 strands x 8 strands, and may be, for example, a symmetric combination such as a combination of 4 strands x 4 strands or a combination of 2 strands x 2 strands, or an asymmetric combination such as a combination of 4 strands x 8 strands or a combination of 2 strands x 4 strands. The cross-sectional shape of the strands in the braid 56 is not particularly limited to the circular shape, and may be substantially rectangular or elliptical. The material of the strands in the braid 56 is not particularly limited, and may be not only metal such as tungsten or stainless steel, but also resin.

An outer periphery of the braid 56 is covered with the outer layer 58 formed of resin. The resin material that forms the outer layer 58 is not particularly limited, and may be, for example, polyamide, polyamide elastomer, polyester, or polyurethane.

A distal end portion of the inner shaft 50 has an extending portion 52 extending from the distal end of the distal-end outer shaft part 31. A radiopaque marker 70 is attached to an outer periphery of the extending portion 52. As the marker 70, a ring formed of a radiopaque material, such as platinum or tungsten, or a coil formed by winding a radiopaque metal wire. The manipulator can grasp the position of the balloon 20 in the blood vessel from the marker 70.

A distal end of the extending portion 52 of the inner shaft 50 is welded to the distal-end attachment part 22 of the balloon 20 with a tip 60 being disposed therebetween. The tip 60 has a tapered outer shape with an outer diameter gradually decreasing toward a distal end thereof, and is formed of flexible resin. The resin material of the tip 60 is not particularly limited, and for example, may be polyurethane or polyurethane elastomer.

The tip 60 is a cylindrical member that forms a distal end portion of the guide wire lumen 51, and has a distal-end guide wire port 69 at a distal end thereof.

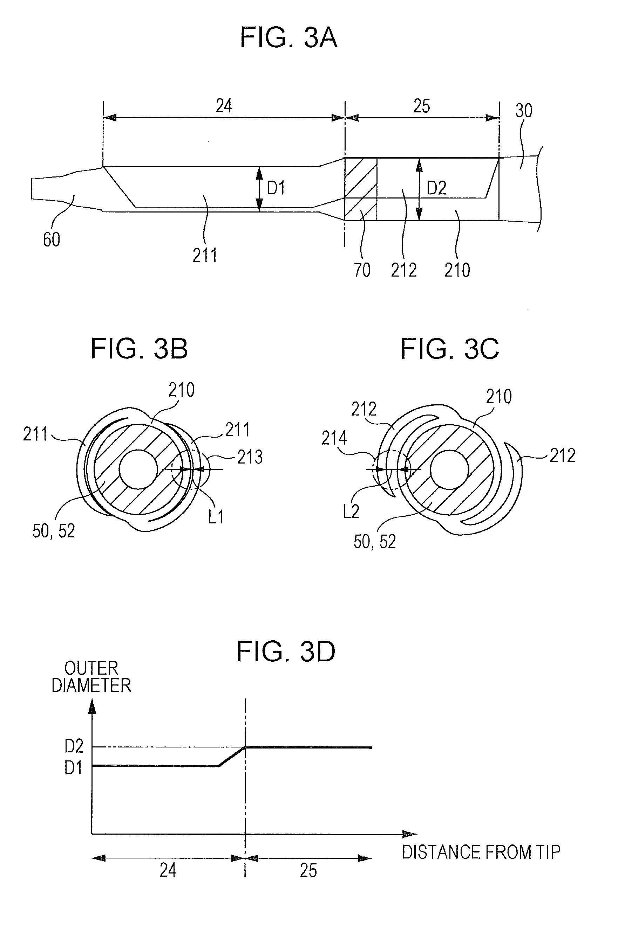

Next, a folded state of the balloon 20 will be described with reference to Figs. 3A to 3D.

When the balloon 20 is folded under reduced pressure, it is divided into a body part 210 that covers the outer periphery of the extending portion 52 of the inner shaft 50 and wing parts 211 and 212 wound around the outer periphery of the body part 210 (see Figs. 3B and 3C). As will be described below, different heat-shrinkable tubes are used in a distal end section 24 of the balloon 20 (from the distal-end attachment part 22 to the marker 70) and a proximal end section 25 of the balloon 20 (from the marker 70 to the proximal-end attachment part 23). More specifically, the distal end section 24 of the balloon 20 is more strongly contracted than the proximal end section 25 of the balloon 20. For this reason, the folding degree of the wing parts 211 in the distal end section 24 and the folding degree of the wing parts 212 in the proximal end section 25 are different from each other. Therefore, a gap 213 (gap length: L1) between the body part 210 and the wing parts 211 in the distal end section 24 is narrower than a gap 214 (gap length: L2) between the body part 210 and the wing parts 212 in the proximal end section 25 (L1 < L2).

Since the distal end section 24 is strongly folded, an outer diameter D1 of the distal end section 24 is small. In contrast, since the proximal end section 25 is weakly folded, an outer diameter D2 of the proximal end section 25 is large (D1 < D2) (see Figs. 3A and 3D).

While the number of each of the wing parts 211 and 212 is two in the balloon 20 in Figs. 3A to 3D, the number of wing parts is not limited thereto, and may be increased. While the wing parts 211 and 212 of the balloon 20 are wound around the body part 210 in a counterclockwise direction (left-handed direction), they may be wound in a clockwise direction (right-handed direction). Alternatively, wing parts 211 and 212 on one side may be wound in the counterclockwise direction (left-handed direction) and wing parts 211 and 212 on the other side may be wound in the clockwise direction (right-handed direction) such as to overlap with each other.

In this way, the outer diameter D1 is small and the gap 213 between the body part 210 and the wing parts 211 is narrow in the distal end section 24. In contrast, the outer diameter D2 is large and the gap 214 between the body part 210 and the wing parts 212 is wide in the proximal end section 25. Hence, the narrowed part can be dilated by the proximal end section 25 with a large diameter by pushing in the balloon catheter 10 with the distal end section 24 having a small outer diameter being caught by the narrowed part. Further, since the gap 214 between the body part 210 and the wing parts 212 in the proximal end section 25 is wide, liquid for inflating the balloon 20 can easily flow into the balloon 20, and this can increase the inflation speed. Still further, since the balloon 20 stores the shape such that the gap between the body part 210 and the wing parts 211 is narrow in the distal end section 24, when the balloon 20 is returned from an inflated state (a state of Fig. 2) to a folded state (a state of Fig. 3A) after medical treatment, the wing parts 211 of the distal end section 24 quickly return to a position where the gap 213 to the body part 210 is narrow. This allows the liquid to be swiftly collected from the distal end of the balloon 20, and therefore, increases the deflation speed.

To make the gap 213 narrower than the gap 214, the wing parts 211 are set in contact with the outer periphery of the body part 210 in the distal end section 24 (see Fig. 3B), and the wing parts 212 are separated from the outer periphery of the body part 210 in the proximal end section 25 (see Fig. 3C). Alternatively, it may be possible to adopt a structure such that the wing parts 211 bite the body part 210 in the distal end section 24 and the wing parts 212 are in contact with the outer periphery of the body part 210 in the proximal end section 25.

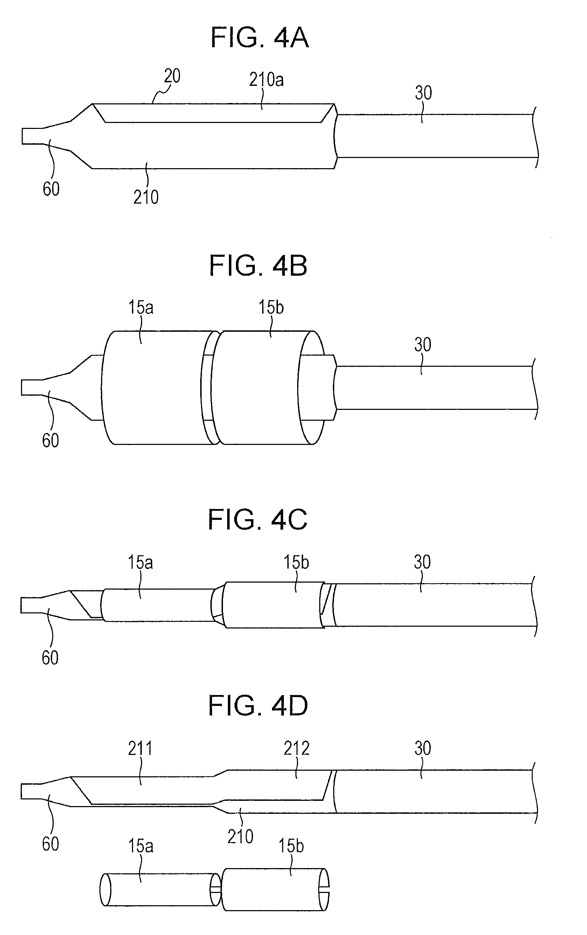

Next, a process for folding the balloon 20 will be described with reference to Figs. 4A to 4D.

When the balloon 20 is folded under reduced pressure, the body part 210 covering the outer periphery of the extending portion 52 of the inner shaft 50 and a wing part 210a wound around the outer periphery of the body part 210 are formed (see Fig. 4A). A distal end of the balloon 20 (in other words, a portion that is to finally become the distal end section 24) is covered with a heat-shrinkable tube 15a having high shrinkability, and a proximal end of the balloon 20 (in other words, a portion to finally become the proximal end section 25) is covered with a heat-shrinkable tube 15b having low shrinkability (see Fig. 4B). After that, the heat-shrinkable tubes 15a and 15b are shrunk by a known heater (see Fig. 4C). Finally, when the heat-shrinkable tubes 15a and 15b are removed from the balloon 20, the distal end section 24 having the strongly folded wing parts 211 and the proximal end section 25 having the weakly folded wing parts 212 are formed (see Fig. 4D).

While the different heat-shrinkable tubes 15a and 15b are used in the distal end section 24 and the proximal end section 25 in the embodiment, the present invention is not limited thereto. Alternatively, a single heat-shrinkable tube whose shrinkability is high at a distal end and low at a proximal end may be used.

When there is no need to contract the proximal end of the balloon 20 by the heat-shrinkable tube 15b (in other words, when there is no problem even if the outer diameter D2 of the proximal end section 25 is large), the distal end section 24 having the strongly folded wing parts 211 and the proximal end section 25 having the wing parts 210a, which are not contracted by the heat-shrinkable tube 15b, may be formed by contracting only the distal end of the balloon 20 by the heat-shrinkable tube 15a.

In the above-described embodiment, the marker 70 having rigidity is used at the boundary between the distal end section 24 and the proximal end section 25 (see Fig. 3A). When the marker 70 having the outer diameter D2 larger than the outer diameter of the extending portion 52 of the inner shaft 50 is attached to the outer periphery of the extending portion 52 at the boundary between the distal end section 24 and the proximal end section 25, the distal end section 24 is folded with reference to the outer diameter D1 of the extending portion 52, and the proximal end section 25 is folded with reference to the outer diameter D2 of the marker 70, so that the distal end section 24 can have a small outer diameter and the proximal end section 25 can have a large outer diameter.

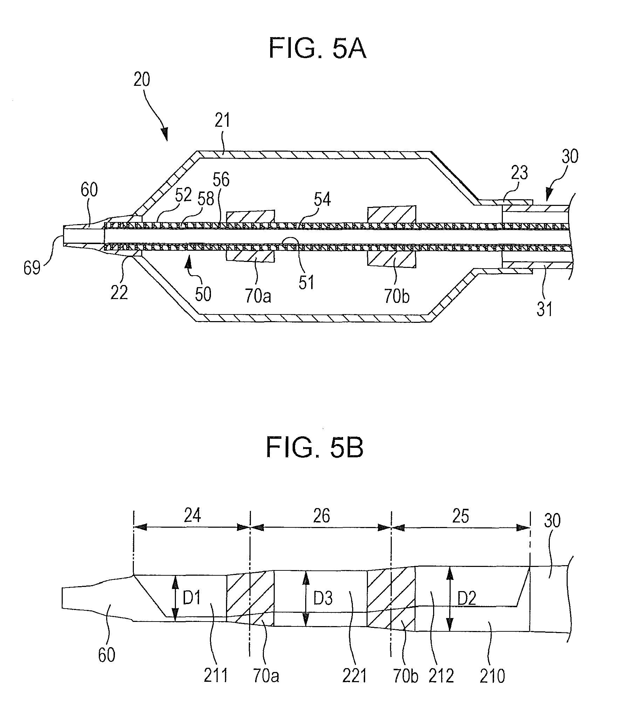

While the single marker 70 is adopted in the embodiment for plain explanation, the present invention is not limited thereto. In another embodiment, as illustrated in Figs. 5A and 5B, a plurality of markers 70 may be used. In this case, a portion from a distal-end attachment part 22 to a distal end marker 70a is regarded as a distal end section 24, a portion from a proximal end marker 70b to a proximal-end attachment part 23 is regarded as a proximal end section 25, and a portion from the distal end marker 70a to the proximal end marker 70b is regarded as a middle section 26, and the folding degree of the balloon 20 is changed among the sections. Thus, the distal end section 24 is strongly folded to have a small diameter D1, the middle section 26 is folded in a medium degree to have an outer diameter D3 that is not small and not large, and the proximal end section 25 is weakly folded to have a large diameter D2 (D1 < D3 < D2). The gap between a body part 210 and wing parts 221 provided in the middle section 26 is wider than a gap 213 formed in the distal end section 24, but is narrower than a gap 214 formed in the proximal end section 25.

By thus setting multiple outer diameters of the balloon 20 in the folded state, the outer diameter does not abruptly change between the outer diameter D1 of the distal end section 24 and the outer diameter D2 of the proximal end section 25. Therefore, when the folded balloon 20 is passed through the narrowed part, the proximal end section 25 having the large outer diameter D2 is restricted from being caught by the narrowed part.

While the ring or coil having a cylindrical shape is used as the marker 70 in the embodiment (see Figs. 2 and 3A), the shape of the marker 70 is not particularly limited. In another embodiment, as illustrated in Figs. 5A and 5B, markers 70a and 70b shaped like a cone with an outer diameter increasing from the distal end toward the proximal end (in other words, a tapered shape with an outer diameter increasing from the distal end toward the proximal end) may be used. In this case, the balloon 20 can be tapered such that the outer diameter increases from the distal end section 24 toward the proximal end section 25 at the positions where the markers 70a and 70b are attached. That is, the outer diameter at the boundary between the distal end section 24 and the proximal end section 25 can be adjusted by the markers 70a and 70b having the tapered shape. Since the outer shape at the boundary between the distal end section 24 and the proximal end section 25 has a tapered shape, when the balloon 20 passes through the narrowed part, the narrowed part can be easily dilated by utilizing the tapered shape.

While the marker 70 having rigidity is attached to the outer periphery of the extending portion 52 of the inner shaft 50 in the balloon catheter 10 of the above-described embodiment, the present invention is not limited thereto. The present invention is also applicable to a balloon catheter in which an inner shaft is doped with a radiopaque material without using any marker.

## Claims

1. A balloon catheter (10) comprising:
a balloon (20);
an outer shaft (30) connected to a proximal end of the balloon (20); and
an inner shaft (50) inserted in the outer shaft (30), extending from a distal end of the outer shaft (30) and connected to a distal end of the balloon (20),
wherein the balloon (20) is folded around an outer periphery of the inner shaft (50), **characterized in that**
the balloon (20) is folded so that it includes a body part (210) covering the outer periphery of the inner shaft (50) and a wing part (211, 212, 221) wound around an outer periphery of the body part (210), and
the folded balloon (20) has a distal end section (24) having a first outer diameter (D1) and a proximal end section (25) having a second outer diameter (D2) larger than the first outer diameter, wherein a radial gap (213) between the body part (210) and the wing part (211, 212, 221) in the distal end section (24) is less than a radial gap (214) between the body part (210) and the wing part (211, 212, 221) in the proximal end section (211, 212, 221).

2. The balloon catheter according to Claim 1, wherein the distal end section (24) is more strongly folded than the proximal end section (25).

3. The balloon catheter according to Claim 1 or 2,
wherein in the distal end section (24) the wing part (211, 212, 221) is strongly folded and in the proximal end section (25) the wing part (211, 212, 221) is weakly folded.

4. The balloon catheter according to any one of Claims 1 to 3, wherein the distal end of the balloon (20) is covered with a heat-shrinkable tube (15a) having high shrinkability, and the proximal end of the balloon (20) is covered with a heat shrinkable tube (15b) having low shrinkability.

5. The balloon catheter according to any one of Claims 1 to 4, further comprising:
a member (70, 70a, 70b) provided on an outer periphery of the inner shaft (50) at a boundary between the distal end section (24) and the proximal end section (25), and having an outer diameter larger than an outer diameter of the inner shaft (50).

6. The balloon catheter according to Claim 5, wherein the member (70) has a tapered shape with the outer diameter increasing in a direction from the distal end section (24) toward the proximal end section (25).

7. The balloon catheter according to Claim 5 or 6,
wherein the member (70) is a marker.

8. A process for folding a balloon (20) of a balloon catheter (10), in particular according to any one of Claims 1 to 7, wherein the balloon catheter (10) comprises:
the balloon (20);
an outer shaft (30) connected to a proximal end of the balloon (20); and
an inner shaft (50) inserted in the outer shaft (30), extending from a distal end of the outer shaft (30) and connected to a distal end of the balloon (20),
wherein the balloon (20) is folded around an outer periphery of the inner shaft (50), **characterized in that**
the balloon (20) is folded so that it is divided into a body part (210) covering the outer periphery of the inner shaft (50) and a wing part (211, 212, 221) wound around an outer periphery of the body part (210), and
a distal end section (24) of the balloon (20) is more strongly folded than a proximal end section (25) of the balloon (20) so that a first outer diameter (D1) of the distal end section (24) is larger than a second outer diameter (D2) of the proximal end section (25) and a radial gap (213) between the body part (210) and the wing part (211, 212, 221) in the distal end section (24) is less than a radial gap (214) between the body part (210) and the wing part (211, 212, 221) in the proximal end section (211, 212, 221).
